# EUROPEAN PATENT APPLICATION

(11) **EP 2 055 790 A1**
(43) Date of publication of application: **06.05.2009**
(21) Application number: 07021202.2
(22) Date of filing: 30.10.2007
(51) Int. Cl.: C12Q 1/68

(54) **Calibration molecule and method for determining the number of copies of genes/target loci in a genome using said calibration molecule**

(71) Applicant: Leibniz-Institut für Altersforschung - Fritz-Lipmann-Institut e.V., 07745 Jena (DE)
(72) Inventor: Huse, Klaus, 04416 Markleeberg (DE); Müller, Oliver, 07745 Jena (DE); Groth, Marco, 07743 Jena (DE); Taudien,Stefan, 07743 Jena (DE); Platzer, Matthias, 07743 Jena (DE); Rosenstiel, Philip, 24116 Kiel (DE); Schreiber, Stefan, 24105 Kiel (DE)
(74) Representative: Krauss, Jan

(57) **Abstract**

The present invention relates to a calibration molecule (CM) and a method for determining the number of copies of genes/target loci in a genome using said CM. Said method comprises the steps of a) providing a sample containing nucleic acids comprising said target locus and a calibration locus with a known copy number, b) providing a calibrator nucleic acid, wherein said calibrator nucleic acid contains at least one modified target locus and at least one modified calibration locus in a predetermined ratio that are both suitable for amplification, wherein said modification is an identifiable difference in the nucleotide sequence of said modified target locus and modified calibration locus, compared to the target locus and a calibration locus, c) admixing said sample with said calibrator nucleic acid, d) amplifying said target locus and modified target locus, and said calibration locus and modified calibration locus, e) determining the ratio of said target locus and modified target locus as amplified in the mixture, and the ratio of said calibration locus and modified calibration locus as amplified in the mixture, and f) determining the copy number of said target locus in said sample based on the ratios as determined in step e).

## Description

The present invention relates to a calibration molecule (CM) and a method for determining the number of copies of genes/target loci in a genome using said CM.

Gene duplication or amplification is one of the key factors driving genetic innovation, i.e., producing novel genetic variants. Although the contribution of whole-genome and segmental duplications to phenotypic diversity across species is widely appreciated, the phenotypic spectrum and potential pathogenicity of small-scale duplications in individual genomes are less well explored. Thus, the correlation of the number of copies of a gene with diseases is of growing interest for research, prognosis, diagnosis, and therapy. Recent data proposes that this mechanism of changing the number of genes is of general medical relevance.

Conrad and Antonarakis (in: Gene Duplication: A Drive for Phenotypic Diversity and Cause of Human Disease. Annu Rev Genomics Hum Genet. 2007 Mar 26) discuss the nature of segmental duplications and the phenotypes produced by such duplications. Phenotypic variation and disease phenotypes induced by duplications are a major class of disease-related genomic variation. Pathogenic duplications particularly involve dosage-sensitive genes with both similar and dissimilar over- and underexpression phenotypes, and genes encoding proteins with a propensity to aggregate. Phenotypes related to human-specific copy number variation in genes regulating environmental responses and immunity are increasingly recognized.

Lee and Lupski, for example, describe a link of genomic rearrangements and gene copy-number alterations with nervous system disorders (Genomic rearrangements and gene copy-number alterations as a cause of nervous system disorders. Neuron. 2006 Oct 5;52(1):103-21.).

WO 98/30883 describes a method of determining copy number of a gene present in an individual, comprising: analyzing a plurality of polymorphic sites in a chromosome containing a gene from an individual to determine the number of different polymorphic forms present at each site; and assigning the copy number of the gene as the highest number of polymorphic forms present at a single site. The methods are described as particularly useful for analysis of biotransformation genes, such as cytochromes P450.

Armour et al. (in: Accurate, high-throughput typing of copy number variation using paralogue ratios from dispersed repeats. Nucleic Acids Res. 2006 Dec 14) describe a comparative PCR method based on dispersed repeat sequences, using a single pair of precisely designed primers to amplify products simultaneously from both test and reference loci, which are subsequently distinguished and quantified via internal sequence differences. The method is described as suitable for copy number analysis.

Hollox et al. (in: Extensive normal copy number variation of a beta-defensin antimicrobial gene cluster Am J Hum Genet. 2003 Sept; 73; 73(3); 591-600) describe the use of a combination of multiplex amplifiable probe hybridization and semiquantitative fluorescence in situ hybridization (SQ-FISH) for the analysis of the DNA copy number variation across chromosome band 8p23.1, a region that is frequently involved in chromosomal rearrangements.

All the above methods suffer from specific drawbacks, such as the dependency from the quality of amplification reactions, hybridizations, enzymatic color formation, the requirement for exact preparation of chemical solutions, and the like.

It is therefore an object of the present invention to provide a method for determining the number of copies of target loci in a nucleic acid sample, such as a genomic sample that is robust, cheap, and reliable, and in particular allows a result that is independently from the quality of the amplification reaction. This method should be also applicable in diagnostics and in a high-throughput environment.

This object of the present invention in a first aspect thereof is solved by a method for determining the copy number of a target locus in a sample containing nucleic acids comprising said target locus, wherein said method comprises the steps of a) providing a sample containing nucleic acids comprising said target locus in an unknown number of copies, and a calibration locus with a known copy number, b) providing a calibrator nucleic acid, wherein said calibrator nucleic acid contains at least one modified target locus and at least one modified calibration locus in a predetermined ratio, that are both suitable for amplification, wherein said modification is an identifiable difference in the nucleotide sequence of said modified target locus and modified calibration locus, compared to the target locus and a calibration locus, c) admixing said sample with said calibrator nucleic acid, d) amplifying said target locus and modified target locus, and said calibration locus and modified calibration locus, e) determining the ratio of said target locus and modified target locus as amplified in the mixture, and the ratio of said calibration locus and modified calibration locus as amplified in the mixture, and f) determining the copy number of said target locus in said sample based on the ratios as determined in step e).

In a preferred embodiment of the method according to the present invention, the design of the calibrator nucleic acid is chosen in such a way that the sample to be examined has merely to be admixed with two oligonucleotide primers (such as DNA, RNA or PNA primers) that are matching with the loci as present on the calibrator nucleic acid, and the number of copies of the gene/target locus in question can be determined by sequencing in relation to a gene/calibrator locus having a known and constant number of copies.

The calibrator nucleic acid to be used in the method according to the present invention can be any nucleic acid. In a preferred method according to the present invention, said nucleic acid sequence is selected from DNA, RNA, cDNA, and in particular genomic DNA.

The predetermined ratio of said modified target and modified calibration locus to be used in the method according to the present invention can be any suitable ratio. Usually, ratios of the modified target locus and modified calibration locus are selected from 1:2, 2:1, and 1:1. The calibrator nucleic acid could also comprise two or more modified target and/or modified calibration loci, in order to be used in order to determine the copy number of more than one locus in the sample at the same time. In this case, different or identical primers can be used for the amplification. That is to say that the design of the calibrator nucleic acid is flexible with respect to its contemplated use for detection.

Again depending from the contemplated use, the calibrator nucleic acid can be any nucleic acid, such as for example a single stranded or double stranded DNA-fragment, in particular a plasmid or a *de novo* synthesized oligonucleotide. Preferred is a method according to the present invention, wherein said calibrator nucleic acid is a single stranded or double stranded DNA-fragment, in particular a plasmid or a *de novo* synthesized oligonucleotide. Depending from the actual circumstances of the analysis, also several fragments or several calibrator nucleic acids can be used, as long as the ratio of said modified target loci to be used in the method according to the present invention is controlled (predetermined), and thus known.

In order to be identifiable, the modified target locus and modified calibration locus (or loci, respectively) on the calibrator nucleic acid(s) carry a modification in their sequence that provides an identifiable difference in the nucleotide sequence of said modified target locus and modified calibration locus, compared to the respective target locus and a calibration locus. In principle, said modification can be any mutation in the sequence that allows for a convenient and reliable identification and does not interfere with the later amplification. Usually, said modification is selected from a mutation, inversion, deletion, in particular a single nucleotide exchange, such as G to C or A to T. Furthermore, modified bases could be used, as long as they give a difference in the later amplification. Preferred is a method according to the present invention, wherein said modification is selected from a mutation, inversion, deletion, in particular a single nucleotide exchange.

For amplification, the sample to be analyzed (wherein the nucleic acid to be analyzed can be present partially or fully purified) is admixed with the calibrator nucleic acid (forming the "mixture"), and subjected to amplification. In the context of the present invention, any suitable method for the amplification of the loci as analyzed might be employed. The amplification method(s) can also include the use of labeled primers, such as primers labeled with a fluorescent and/or enzymatic label. Preferred is a method according to the present invention, wherein said amplification comprises polymerase chain reaction (PCR).

The determining of the ratio(s) of the target loci and modified target loci and/or the ratio(s) of said calibration loci and modified calibration loci as amplified in the mixture comprises a method that allows for a quantitative detection of the different amplification products of the loci as present in the mixture. Preferred are restriction digestion (using cleaving that is based on the modification differences of the fragments, and the detection of the length and/or weight of the fragments as generated, and/or sequencing, in particular pyrosequencing.

As stated above, it is known that the copy number of genes has implications in many medical and/or genetic conditions, such as, for example, the copy number of an oncogene and the out-brake and/or progression of cancer or metastasis. Another important aspect of the present invention therefore relates to a method according to the present invention that further comprises the step of diagnosing a disease, wherein said diagnosis is at least based in part on the copy number of the target locus (or loci) as determined in said sample. Preferred is a method according to the present invention, wherein the copy number is elevated or reduced, compared to the copy number of said gene in a sample that is derived from a healthy person.

The disease to be diagnosed can be selected from any disease that is known to be associated (related) with/to a specific copy number of a target genetic locus (or loci). Preferred is a method according to the present invention, wherein said disease is selected from a neuronal and/or proliferative disease, such as cancer and/or inflammation.

Another important aspect of the present invention then relates to a calibrator nucleic acid for a specific target locus, comprising at least one modified target locus and at least one modified calibration locus in a predetermined ratio, wherein both loci are suitable for amplification, wherein said modification is an identifiable difference in the nucleotide sequence of said modified target locus and modified calibration locus, compared to the target locus and a calibration locus.

The calibrator nucleic acid to be used in the method according to the present invention can be any nucleic acid. A preferred calibrator nucleic acid according to the present invention is selected from DNA, RNA, cDNA, and in particular genomic DNA.

The predetermined ratio of said modified target and modified calibration locus in the calibrator nucleic acid according to the present invention can be any suitable ratio. Usually, ratios of the modified target locus and modified calibration locus are selected from 1:2, 2:1, and 1:1. The inventive calibrator nucleic acid could also comprise two or more modified target and/or modified calibration loci, in order to be used in order to determine the copy number of more than one locus in the sample at the same time. In this case, different or identical primers can be used for the amplification. That is to say that the design of the calibrator nucleic acid is flexible with respect to its contemplated use for detection.

Again depending from the contemplated use, the calibrator nucleic acid can be any nucleic acid, such as for example a single stranded or double stranded DNA-fragment, in particular a plasmid or a *de novo* synthesized oligonucleotide. Preferred is a calibrator nucleic acid according to the present invention, wherein said calibrator nucleic acid is a single stranded or double stranded DNA-fragment, in particular a plasmid or a *de novo* synthesized oligonucleotide. Depending from the actual circumstances of the analysis, also several fragments or several calibrator nucleic acids can be used, as long as the ratio of said modified target loci to be used in the method according to the present invention is controlled (predetermined), and thus known.

In order to be identifiable, the modified target locus and modified calibration locus (or loci, respectively) on the calibrator nucleic acid(s) carry a modification in their sequence that provides an identifiable difference in the nucleotide sequence of said modified target locus and modified calibration locus, compared to the respective target locus and a calibration locus. In principle, said modification can be any mutation in the sequence that allows for a convenient and reliable identification and does not interfere with the later amplification. Usually, said modification is selected from a mutation, inversion, deletion, in particular a single nucleotide exchange, such as G to C or A to T. Furthermore, modified bases could be used, as long as they give a difference in the later amplification. Preferred is a calibrator nucleic acid according to the present invention, wherein said modification is selected from a mutation, inversion, deletion, in particular a single nucleotide exchange.

Another important aspect of the present invention then relates to a kit for performing a method according to the present invention, wherein said kit comprises a calibrator nucleic acid according to the present invention, optionally with other components that are to be used in the methods of the present invention, such as, for example, primers, buffers, instructions for use, and/or other components.

Finally, another important aspect of the present invention then relates to a method for preventing, treating and/or monitoring a disease that relates to the copy number of the target locus (or loci), comprising the method according to the present invention, and treating the disease at least in part on the copy number of the target locus (or loci) as determined in said sample. Preferred is such method according to the present invention, wherein the copy number is elevated or reduced, compared to the copy number of said gene in a sample that is derived from a healthy person. The diseases that can be treated, monitored and/or prevented are the same as mentioned above.

The present invention will now be described in more detail in the following examples with reference to the accompanying Figures, without being limited thereto. For the purposes of the present invention, all references as cited herein are incorporated by reference in their entireties. In the Figures

Fig. 1 shows the alignment of the calibrator DNA (designated calib 1) with the genomic sequences of exon 4 of the *NOD2* gene on chromosome 16 (one copy per haploide genome), of exon 2 of the CNV-affected *DEFB4* gene on chromosome 8 (variable copy number).

Fig. 2 shows the outcome of the pyrosequencing of genomic DNA (Figure 2A and B), and the mixture of the genomic DNA with the calibrator DNA (Figure 2C and D). The ratios of the informative nucleotides allows for the calculation of the copy numbers. Figures 2A and C are for *NOD2,* and Figures 2B and D are for *DEFB4* using sequencing primer adjacent to the modified nucleotide positions. The informative position is marked.

### Example

Determination of the copy number of the gene *DEFB4*
With reference to Figure 1, a calibrator nucleic acid was developed that contained a *NOD2-*modified target locus including a "C"-mutation as a modification, compared to the "T"-chromosomal position in the NOD2-exon 4. After amplification of the calibrator nucleic acid with the genomic DNA sample, a ratio of T/C of 0.84 was found for the single copy gene *NOD2.*

In addition, the calibrator nucleic acid contained a *DEFB4*-modified target locus including a "G"-mutation as a modification, compared to the "C"-chromosomal position in the *DEFB4-*exon 2. After amplification of the calibrator nucleic acid with the genomic DNA sample, a ratio of C/G of 2.1 was found.

Therefore, a copy number of 5 per diploid genome could be calculated (2.1/0.84 = 2.5)

## Claims

1. A method for determining the copy number of a target locus in a sample containing nucleic acids comprising said target locus, comprising the steps of
a) providing a sample containing nucleic acids comprising said target locus and a calibration locus with a known copy number,
b) providing a calibrator nucleic acid, wherein said calibrator nucleic acid contains at least one modified target locus and at least one modified calibration locus in a predetermined ratio that are both suitable for amplification, wherein said modification is an identifiable difference in the nucleotide sequence of said modified target locus and modified calibration locus, compared to the target locus and a calibration locus,
c) admixing said sample with said calibrator nucleic acid,
d) amplifying said target locus and modified target locus, and said calibration locus and modified calibration locus,
e) determining the ratio of said target locus and modified target locus as amplified in the mixture, and the ratio of said calibration locus and modified calibration locus as amplified in the mixture, and
f) determining the copy number of said target locus in said sample based on the ratios as determined in step e).

2. The method according to claim 1, wherein said nucleic acid is selected from DNA, RNA, cDNA, and genomic DNA.

3. The method according to claim 1 or 2, wherein said predetermined ratio of said modified target locus and modified calibration locus is selected from 1:2, 2:1, and 1:1.

4. The method according to any of claims 1 to 3, wherein said calibrator nucleic acid is a single stranded or double stranded DNA-fragment, in particular a plasmid or an *in silico* synthesized oligonucleotide.

5. The method according to any of claims 1 to 4, wherein said modification is selected from a mutation, inversion, deletion, in particular a single nucleotide exchange.

6. The method according to any of claims 1 to 5, wherein said amplification comprises PCR.

7. The method according to any of claims 1 to 6, wherein said determining the ratio of said target loci and modified target loci and/or the ratio of said calibration loci and modified calibration loci as amplified in the mixture comprises restriction digestion and/or sequencing, in particular pyrosequencing.

8. The method according to any of claims 1 to 7, wherein said target locus is an oncogene or any other disease gene.

9. The method according to any of claims 1 to 8, further comprising diagnosing a disease, at least based in part on said copy number of said target locus as determined in said sample.

10. The method according to any of claims 1 to 9, wherein the copy number is elevated or reduced.

11. The method according to any of claims 1 to 10, wherein said disease is a neuronal or proliferative disease, such as cancer or inflammation.

12. A calibrator nucleic acid for a specific target locus, comprising at least one modified target locus and at least one modified calibration locus in a predetermined ratio that are both suitable for amplification, wherein said modification is an identifiable difference in the nucleotide sequence of said modified target locus and modified calibration locus, compared to the target locus and a calibration locus.

13. The calibrator nucleic acid according to claim 12, wherein said nucleic acid is selected from DNA, RNA, cDNA, and genomic DNA.

14. The calibrator nucleic acid according to claim 12 or 13, wherein said predetermined ratio of said modified target locus and modified calibration locus is selected from 1:2, 2:1, and 1:1.

15. The calibrator nucleic acid according to any of claims 12 to 14, wherein said calibrator nucleic acid is a single stranded or double stranded DNA-fragment, in particular a plasmid or a *in silico* synthesized oligonucleotide.

16. The calibrator nucleic acid according to any of claims 12 to 15, wherein said modification is selected from a mutation, inversion, deletion, in particular a single nucleotide exchange.
